# EUROPEAN PATENT SPECIFICATION

(11) **EP 3 518 905 B1**
(45) Date of publication and mention of the grant of the patent: **28.04.2021**
(21) Application number: 17794060.8
(22) Date of filing: 29.09.2017
(51) Int. Cl.: A61K 9/20, A61K 31/496

(54) **PHARMACEUTICAL COMPOSITIONS OF 5-HT6 ANTAGONIST**
PHARMAZEUTISCHE ZUSAMMENSETZUNGEN EINES 5-HT6-ANTAGONISTEN
COMPOSITIONS PHARMACEUTIQUES D'ANTAGONISTE DE 5-HT6

(30) Priority: 03.10.2016 IN 201641033741
(43) Date of publication of application: 07.08.2019
(73) Proprietor: Suven Life Sciences Limited, Hyderabad, Telangana 500034 (IN)
(72) Inventor: NIROGI, Ramakrishna, Hyderabad Telangana 500034 (IN); MUDIGONDA, Koteshwara, Hyderabad Telangana 500034 (IN); DOGIPARTI, Dhanunjay Kumar, Hyderabad Telangana 500034 (IN); JASTI, Venkateswarlu, Hyderabad Telangana 500034 (IN)
(74) Representative: HGF
(86) International application number: PCT/IB2017/056009
(87) International publication number: WO 2018/065869

(56) References cited:
- WO-A1-2015/083179
- US-B2- 7 875 605

## Description

### Field of Invention

The present invention relates to immediate release (IR) pharmaceutical compositions comprising 1-[(2-bromophenyl)sulfonyl]-5-methoxy-3-[(4-methyl-1-piperazinyl)methyl]-1H-indole or a pharmaceutically acceptable salt (s) thereof as an active ingredient and one or more pharmaceutically acceptable excipients and to methods of preparation of said compositions.

### Background of Invention

Alzheimer's disease (AD) is the most common cause of dementia worldwide. The exponential rise in the number of cases of AD in the past and the future projection over the next few decades is anticipated to result in great pressure on the social and health-care systems of developed and developing economies alike. AD also imposes tremendous emotional and financial burden to the patient's family and community.

The compound of the present invention is a pure 5-hydroxytryptamine 6 receptor (5-HT₆R) antagonist with high affinity and very high selectivity over closely related serotonin receptor subtypes and improves learning and memory in animals. The 5-HT₆R antagonist, 1-[(2-bromophenyl)sulfonyl]-5-methoxy-3-[(4-methyl-1-piperazinyl)methyl]-1H-indole or a pharmaceutically acceptable salt (s) thereof is described in US7875605 which is incorporated by reference.

1-[(2-Bromophenyl)sulfonyl]-5-methoxy-3-[(4-methyl-1-piperazinyl)methyl]-1H-indole dimesylate monohydrate (herein after referred to as Compound 1), which has chemical structure, 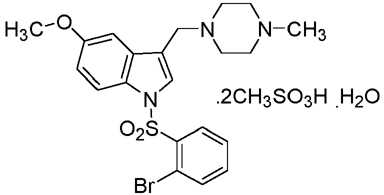 is a promising pharmaceutical active agent intended for the symptomatic treatment of Alzheimer's disease and other disorders of memory and cognition like Attention deficient hyperactivity, Parkinson's disease, schizophrenia, lewy body dementia, vascular dementia or frontotemporal dementia. The process for preparing compound 1 on a larger scale is described in WO2015083179A1.

There is a need to develop a suitable dosage form of the compound 1 to treat the patients with AD and other disorders of memory and cognition like Attention deficient hyperactivity, Parkinson's disease, schizophrenia, lewy body dementia, vascular dementia or frontotemporal dementia. In our present invention, we developed IR pharmaceutical compositions of compound 1 having (1) excellent properties of tablet formation, (2) excellent wetting, disintegration, rapid and complete drug release properties, (3) good purity profile and (4) stable formulation for the treatment of AD and other disorders of memory and cognition like Attention deficient hyperactivity, Parkinson's disease, schizophrenia, lewy body dementia, vascular dementia or frontotemporal dementia.

### Summary of Invention

In one aspect, the present invention relates to immediate release pharmaceutical composition that on a total of 100% by weight comprises:
a) from 2 % to 60 % 1-[(2-bromophenyl)sulfonyl]-5-methoxy-3-[(4-methyl-1-piperazinyl)methyl]-1H-indole or a pharmaceutically acceptable salt thereof;
b) from 36 % to 97 % diluent or total of two diluents; wherein the diluent is selected from the group consisting of microcrystalline cellulose, lactose monohydrate, dibasic calcium phosphate, lactose, lactose hydrate, lactose anhydrate, mannitol, starch and isomalt;
c) from 0.5 % to 2 % lubricant; wherein the lubricant is magnesium stearate;
d) from 0.5 % to 1 % glidant; wherein the glidant is colloidal silicon dioxide;
e) 0 % to 10 % binder; wherein the binder is selected from group consisting of povidone or hydroxypropyl methylcellulose;
f) 0 % to 5 % disintegrant; wherein the disintegrant is selected from crospovidone, sodium starch glycolate and croscarmellose sodium; and
g) 0 % to 2 % acidifying agent; wherein the acidifying agent is citric acid.

In another aspect, the present invention relates to immediate release pharmaceutical composition as defined above comprising 1-[(2-bromophenyl)sulfonyl]-5-methoxy-3-[(4-methyl-1-piperazinyl)methyl]-1H-indole dimesylate monohydrate.

In another aspect, the present invention relates to the above immediate release pharmaceutical composition comprising 1-[(2-bromophenyl)sulfonyl]-5-methoxy-3-[(4-methyl-1-piperazinyl)methyl]-1H-indole dimesylate monohydrate, wherein the pharmaceutical composition comprises binder, diluent, lubricant, glidant, disintegrant and acidifying agent.

In yet another aspect, the present invention relates to the above immediate release pharmaceutical composition of 1-[(2-bromophenyl)sulfonyl]-5-methoxy-3-[(4-methyl-1-piperazinyl) methyl]-1H-indole dimesylate monohydrate, wherein said composition comprises on a total of 100 % by weight:
(a) from 2 % to about 3 % 1-[(2-bromophenyl)sulfonyl]-5-methoxy-3-[(4-methyl-1-piperazinyl)methyl]-1H-indole dimesylate monohydrate;
(b) about 95 % to 97 % diluent;
(c) about 1 % lubricant; and
(d) 0.5 % glidant.

In yet another aspect, the present invention relates to the above immediate release pharmaceutical composition of 1-[(2-bromophenyl)sulfonyl]-5-methoxy-3-[(4-methyl-1-piperazinyl)methyl]-1H-indole dimesylate monohydrate, wherein said composition comprises on a total of 100 % by weight:
(a) from about 11 % to about 38 % 1-[(2-bromophenyl)sulfonyl]-5-methoxy-3-[(4-methyl-1-piperazinyl)methyl]-1H-indole dimesylate monohydrate;
(b) from about 61 % to about 87 % of one diluent or total of two diluents;
(c) about 1 % lubricant;
(d) 0.5 % glidant;
(e) about 2 % disintegrant; and
(f) about 1 % acidifying agent.

In yet another aspect, the present invention relates to the above immediate release pharmaceutical composition of 1-[(2-bromophenyl)sulfonyl]-5-methoxy-3-[(4-methyl-1-piperazinyl)methyl]-1H-indole dimesylate monohydrate, wherein said composition comprises on a total of 100 % by weight:
(a) from about 24 % to about 38 % 1-[(2-bromophenyl)sulfonyl]-5-methoxy-3-[(4-methyl-1-piperazinyl)methyl]-1H-indole dimesylate monohydrate;
(b) from about 61 % to about 72 % one diluent or total of two diluents;
(c) from about 1 % to about 1.25 % lubricant;
(d) 0.5 % glidant; and
(e) about 2 % disintegrant.

In yet another aspect, the present invention relates to the above immediate release pharmaceutical composition of 1-[(2-bromophenyl)sulfonyl]-5-methoxy-3-[(4-methyl-1-piperazinyl)methyl]-1H-indole dimesylate monohydrate, wherein said composition comprises on a total of 100 % by weight:
(a) from about 37 % to about 51 % 1-[(2-bromophenyl)sulfonyl]-5-methoxy-3-[(4-methyl-1-piperazinyl)methyl]-1H-indole dimesylate monohydrate;
(b) from about 45 % to about 60 % diluent;
(c) about 1 % lubricant;
(d) 0.5 % glidant;
(e) about 2 % of disintegrant; and
(f) about 1 % acidifying agent.

In yet another aspect, the present invention relates to the above immediate release pharmaceutical composition of 1-[(2-bromophenyl)sulfonyl]-5-methoxy-3-[(4-methyl-1-piperazinyl)methyl]-1H-indole dimesylate monohydrate, wherein said composition comprises on a total of 100 % by weight:
(a) from about 36 % to about 60 % 1-[(2-bromophenyl)sulfonyl]-5-methoxy-3-[(4-methyl-1-piperazinyl) methyl]-1H-indole dimesylate monohydrate;
(b) from about 36 % to about 62 % diluent;
(c) from 0.5 % to about 1 % lubricant;
(d) 0.5 % glidant; and
(e) about 2 % disintegrant.

In yet another aspect, the present invention relates to the above immediate release pharmaceutical composition of 1-[(2-bromophenyl)sulfonyl]-5-methoxy-3-[(4-methyl-1-piperazinyl)methyl]-1H-indole dimesylate monohydrate, wherein said composition comprises on a total of 100 % by weight:
(a) from about 11 % to about 38 % 1-[(2-bromophenyl)sulfonyl]-5-methoxy-3-[(4-methyl-1-piperazinyl)methyl]-1H-indole dimesylate monohydrate;
(b) from about 61 % to about 72 % diluent;
(c) about 1 % lubricant;
(d) from about 2 % to 10 % binder;
(e) 0.5 % glidant; and
(f) from about 2 % to 5 % disintegrant.

The present disclosure also relates to methods of preparation of immediate release pharmaceutical compositions.

In yet another aspect the present invention relates to an immediate release tablet, wherein said tablet comprises on a total of 100 % by weight:
(a) from 2 % to 60 % 1-[(2-bromophenyl)sulfonyl]-5-methoxy-3-[(4-methyl-1-piperazinyl) methyl]-1H-indole dimesylate monohydrate;
(b) 36 % 97 % of one diluent or total of two diluents as defined above;
(c) 0 % to 10 % binder as defined above;
(d) from 0.5 % to 2 % lubricant as defined above;
(e) from 0.5 % to 1 % glidant as defined above;
(f) 0 % to 5 % disintegrant as defined above; and
(g) 0 % 2 % acidifying agent as defined above.

In yet another aspect, the present invention relates to the above immediate release tablet, wherein said tablet comprises on a total of 100 % by weight:
(a) from 2 % to about 3 % 1-[(2-bromophenyl)sulfonyl]-5-methoxy-3-[(4-methyl-1-piperazinyl)methyl]-1H-indole dimesylate monohydrate;
(b) about 95 % to 97 % diluent;
(c) about 1 % lubricant; and
(d) 0.5 % glidant.

In yet another aspect, the present invention relates to the above immediate release tablet, wherein the said tablet comprises on a total of 100 % by weight:
(a) from about 11 % to about 38 % 1-[(2-bromophenyl)sulfonyl]-5-methoxy-3-[(4-methyl-1-piperazinyl)methyl]-1H-indole dimesylate monohydrate;
(b) from about 61 % to about 87 % of one diluent or total of two diluents;
(c) about 1 % lubricant;
(d) 0.5 % glidant;
(e) about 2 % disintegrant; and
(f) about 1 % acidifying agent.

In yet another aspect, the present invention relates to the above immediate release tablet, wherein said tablet comprises on a total of 100 % by weight:
(a) from about 24 % to about 38 % 1-[(2-bromophenyl)sulfonyl]-5-methoxy-3-[(4-methyl-1-piperazinyl)methyl]-1H-indole dimesylate monohydrate;
(b) from about 61 % to about 72 % one diluent or total of two diluents;
(c) from about 1 % to about 1.25 % lubricant;
(d) 0.5 % glidant; and
(e) about 2 % disintegrant.

In yet another aspect, the present invention relates to the above immediate release tablet,
wherein said tablet comprises on a total of 100 % by weight:
(a) from about 37 % to about 51 % 1-[(2-bromophenyl)sulfonyl]-5-methoxy-3-[(4-methyl-1-piperazinyl)methyl]-1H-indole dimesylate monohydrate;
(b) from about 45 % to about 60 % diluent;
(c) about 1 % lubricant;
(d) 0.5 % glidant;
(e) about 2 % of disintegrant; and
(f) about 1 % acidifying agent.

In yet another aspect, the present invention relates to the above immediate release tablet,
wherein said tablet comprises on a total of 100 % by weight:
(a) from about 36 % to about 60 % 1-[(2-bromophenyl)sulfonyl]-5-methoxy-3-[(4-methyl-1-piperazinyl)methyl]-1H-indole dimesylate monohydrate;
(b) from 36 % to about 62 % diluent;
(c) from 0.5 % to about 1 % lubricant;
(d) 0.5 % glidant; and
(e) about 2 % disintegrant.

In yet another aspect, the present invention relates to the above immediate release tablet,
wherein said tablet comprises on a total of 100 % by weight:
(a) from about 11 % to about 38 % 1-[(2-bromophenyl)sulfonyl]-5-methoxy-3-[(4-methyl-1-piperazinyl)methyl]-1H-indole dimesylate monohydrate;
(b) from about 61 % to about 72 % diluent;
(c) about 1 % lubricant;
(d) from about 2 % to 10 % binder;
(e) 0.5 % glidant; and
(f) from about 2 % to 5 % disintegrant.

In yet another aspect, the present invention relates to the immediate release pharmaceutical composition of dose ranges from about 5 mg to about 200 mg.

In yet another aspect, the present invention relates to the immediate release pharmaceutical composition, wherein the total weight of the immediate release tablet is from about 100 mg to about 600 mg.

In yet another aspect, the present invention relates to the immediate release pharmaceutical composition, wherein the immediate release pharmaceutical composition comprises,
i) less than 0.5 % of chloro impurity;
ii) less than 0.5 % of unknown impurity;
iii) less than 1 % of total impurity.

In yet another aspect, the present invention relates to the immediate release pharmaceutical composition, wherein the purity of the 1-[(2-bromophenyl)sulfonyl]-5-methoxy-3-[(4-methyl-1-piperazinyl) methyl]-1H-indole dimesylate monohydrate is about 99.3 %.

In yet another aspect, the present invention relates to the immediate release pharmaceutical composition, wherein the 1-[(2-bromophenyl)sulfonyl]-5-methoxy-3-[(4-methyl-1-piperazinyl)methyl]-1H-indole dimesylate monohydrate is released about 85 % to about 100 % within 30 minutes.

### Detailed description of Invention

Unless otherwise stated, the following terms used in the specification and claims have the meanings given below:
The term, "pharmaceutically acceptable excipients" as used herein refers to diluents, disintegrants, binders, lubricants, glidants, polymers, coating agents, solvents, co-solvents, preservatives, wetting agents, thickening agents, antifoaming agents, sweetening agents, flavouring agents, antioxidants, colorants, solubulizers, plasticizer or dispersing agents and the like. The pharmaceutical compositions of the present invention may be formulated in a conventional manner using one or more pharmaceutically acceptable excipients.

The "binder" employed in a composition of the present invention is capable for holding the ingredients together and forming the granules with required mechanical strength. Example of binders includes without limitation, polyvinylpyrrolidone (povidone (PVPK30)), polyethlylene glycol (PEG), saccharides, gelatins, pregelatinized starches, hydroxypropylcellulose, hydroxypropyl methylcellulose (HPMC) and cellulose ethers. In the composition of the invention the binder is selected from povidone or hydroxypropyl methylcellulose.

The "diluent" employed in a composition of the present invention is capable for providing bulkiness to obtain a desired immediate release pharmaceutical composition. Preferred diluents are dibasic calcium phosphate, lactose, lactose hydrate, lactose monohydrate, lactose anhydrate, mannitol microcrystalline cellulose; Avicel, Avicel PH 101, Avicel PH 102 or Avicel PH 103, maize starch, Starcap-1500, Starlac and isomalt.

The "disintegrant" employed in a composition of the present invention is capable of facilitating the breakup of an immediate release pharmaceutical composition prepared from the composition when placed in contact with an aqueous medium. Preferred disintegrants are crospovidone (cross-linked homopolymer of N-vinyl-2-pyrrolidinone, i.e., cross-linked 1-ethenyl-2-pyrrolidinone); and sodium starch glycolate.

The "lubricant" employed in a composition of the present invention is capable of preventing the ingredients from clumping together and from sticking to the apparatus on which it is formed, for example, preventing adherence to the face of the upper punch (picking) or lower punch (sticking) of a compression machine. The lubricant is magnesium stearate.

The "glidant" employed in a composition of the present invention is capable for increase in flow, and is colloidal silicon dioxide (Aerosil).

The "acidifying agent" employed in a composition of the present invention is capable to increase the acidity, and is citric acid.

The "coloring agent" (or "colorant") employed in a composition of the present invention may be one or more compounds which impart a desired color to the composition. Addition of a coloring agent may be used, for example, so that tablets of different potencies may be easily distinguished. Example of coloring agent includes but not limited to beta-carotene, indigo carmine, sunset yellow FCF, tartrazine, brilliant blue FCF, titanium dioxide, quinoline yellow, allura red AC, quinizarine green SS and iron oxides, which are accepted universally.

The "active ingredient" defined in this invention is 1-[(2-bromophenyl)sulfonyl]-5-methoxy-3-[(4-methyl-1-piperazinyl)methyl]-1H-indole dimesylate monohydrate.

The term "about" as used herein refers to a defined range of the value by ± 10 %. For example, about 2 % means 1.8 % to 2.2 %, about 5 % means 4.5 % to 5.5 %, about 10 % means 9 % to 11 % and about 40 % means 36 % to 44 %.

The term, "pharmaceutically acceptable salt" as used herein refers to salts of the active ingredient and are prepared by reaction with the appropriate acid or acid derivative, depending on the particular substituents found on the compounds described herein. The pharmaceutically acceptable salt includes but not limited to dimesylate monohydrate salt, dihydrochloride salt, oxalate salt, tartrate salt and the like. Preferably, the pharmaceutically acceptable salt is dimesylate monohydrate salt and dihydrochloride salt. More preferably, the pharmaceutically acceptable salt is dimesylate monohydrate salt.

The term, "patient" as used herein refers to an animal. Preferably the term "patient" refers to mammal. The term mammal includes animals such as mice, rats, dogs, rabbits, pigs, monkeys, horses and human. More preferably the patient is human.

The term, "immediate release composition" refers to a composition of an active ingredient which disintegrates rapidly and releases greater than 85 % at 30 minutes.

The immediate release pharmaceutical compositions of the present invention can be used for treatment or prevention of Alzheimer's disease and other disorders of memory and cognition like Attention deficient hyperactivity, Parkinson's disease, schizophrenia, lewy body dementia, vascular dementia or frontotemporal dementia. The immediate release pharmaceutical composition of the instant invention can be administered orally, in an effective amount, to a mammalian (especially human) subject to treat or prevent the aforementioned disorders.

The effective dosage of the immediate release pharmaceutical composition comprising 1-[(2-bromophenyl)sulfonyl]-5-methoxy-3-[(4-methyl-1-piperazinyl)methyl]-1H-indole dimesylate monohydrate is about 5 mg to about 200 mg. The immediate release pharmaceutical composition can be administered 1 to 3 times per day, based on condition of the patients. The total weight of immediate release pharmaceutical composition of the present invention is from about 100 mg to 600 mg.

The compound 1 belongs to class I as per BCS classification based on our experimental results and hence particle size of the compound 1 does not effect in the treatment of the patient.

In one embodiment the present invention relates to the immediate release pharmaceutical composition comprising:

| Ingredient | Range (% w/w) | Preferred Range (% w/w) |
|---|---|---|
| Compound 1 (Active ingredient) | 2 - 60 | 10-50 |
| Diluent | 36 - 97 | 40 - 90 |
| Binder | 0 - 10 | 3 - 5 |
| Disintegrant | 0 - 5 | 2 - 4 |
| Lubricant | 0.5 - 2 | 0.5 - 1 |
| Glidant | 0.5 - 1 | 0.5 - 1 |
| Acidifying agent | 0 - 2 | 0 - 1 |

In yet another embodiment, the present invention relates to the immediate release pharmaceutical composition comprises 2 % to about 3 % by weight of 1-[(2-bromophenyl)sulfonyl]-5-methoxy-3-[(4-methyl-1-piperazinyl)methyl]-1H-indole dimesylate monohydrate.

In yet another embodiment, the present invention relates to the immediate release pharmaceutical composition comprises about 10 % to about 40 % by weight of 1-[(2-bromophenyl)sulfonyl]-5-methoxy-3-[(4-methyl-1-piperazinyl)methyl]-1H-indole dimesylate monohydrate.

In yet another embodiment, the present invention relates to the immediate release pharmaceutical composition comprises about 20 % to about 40 % by weight of 1-[(2-bromophenyl)sulfonyl] -5 -methoxy-3 - [(4-methyl-1 -piperazinyl) methyl]-1H-indole dimesylate monohydrate.

In yet another embodiment, the present invention relates to the immediate release pharmaceutical composition comprises about 30 % to about 50 % by weight of 1-[(2-bromophenyl)sulfonyl]-5-methoxy-3-[(4-methyl-1-piperazinyl)methyl]-1H-indole dimesylate monohydrate.

In yet another embodiment, the present invention relates to the immediate release pharmaceutical composition comprises about 30 % to about 60 % by weight of 1-[(2-bromophenyl)sulfonyl]-5-methoxy-3-[(4-methyl-1-piperazinyl)methyl]-1H-indole dimesylate monohydrate.

In yet another embodiment, the present invention relates to the immediate release pharmaceutical composition comprising; about 40 % to about 80 % by weight of diluent

In yet another embodiment, the present invention relates to the immediate release pharmaceutical composition comprising about 70 % to about 90 % by weight of diluent.

In yet another embodiment, the present invention relates to the immediate release pharmaceutical composition comprising about 20 % to about 40 % by weight of diluent.

In yet another embodiment, the present invention relates to immediate release pharmaceutical composition in the form of tablet or capsule.

In yet another embodiment, the present invention relates to an immediate release tablet, wherein the tablet comprises,
(a) from 2 % to 60 % 1-[(2-bromophenyl)sulfonyl]-5-methoxy-3-[(4-methyl-1-piperazinyl)methyl]-1H-indole dimesylate monohydrate;
(b) from 36 % to 97 % of microcrystalline cellulose;
(c) from 0.5 % to 2 % magnesium stearate;
(d) from 0.5 % to 1 % colloidal silicon dioxide;
(e) 0 % to 5 % crospovidone; and
(f) 0 % to 2 % citric acid.

In yet another embodiment, the present invention relates to an immediate release tablet, wherein the tablet comprises,
(a) from 2 % to 60 % 1-[(2-bromophenyl)sulfonyl]-5-methoxy-3-[(4-methyl-1-piperazinyl)methyl]-1H-indole dimesylate monohydrate;
(b) from 36 % to 97 % of microcrystalline cellulose;
(c) from 0.5 % to 2 % magnesium stearate;
(d) from 0.5 % to 1 % colloidal silicon dioxide;
(e) 0 % to 10 % povidone;
(f) 0 % to 5 % crospovidone; and
(g) 0 % to 2 % citric acid.

In yet another embodiment, the present invention relates to the immediate release pharmaceutical composition comprises about 40 % to about 80 % by weight of microcrystalline cellulose.

In yet another embodiment, the present invention relates to the immediate release pharmaceutical composition comprises about 70 % to about 90 % by weight of microcrystalline cellulose.

In yet another embodiment, the present invention relates to the immediate release pharmaceutical composition comprises about 20 % to about 40 % by weight of microcrystalline cellulose.

In yet another embodiment, the present invention relates to the immediate release pharmaceutical composition comprises about 2 % by weight of crospovidonc.

In yet another embodiment, the present invention relates to the immediate release pharmaceutical composition comprises about 1 % by weight of citric acid.

In yet another embodiment, the present invention relates to the immediate release pharmaceutical composition comprises about 4 % by weight of povidone.

In other aspect, the present invention relates to the immediate release pharmaceutical composition comprising 1-[(2-bromophenyl)sulfonyl]-5-methoxy-3-[(4-methyl-1-piperazinyl) methyl]-1H-indole or a pharmaceutically acceptable salt thereof for use in the treatment of Alzheimer's disease, memory and cognition disorders selected from Attention deficient hyperactivity disorder, Parkinson's disease, schizophrenia, lewy body dementia, vascular dementia or frontotemporal dementia.

### Methods of preparation of immediate release pharmaceutical composition

Also disclosed is the process for the preparation of the immediate release pharmaceutical composition comprising 1-[(2-bromophenyl)sulfonyl]-5-methoxy-3-[(4-methyl-1-piperazinyl)methyl]-1H-indole or a pharmaceutically acceptable salt thereof.

The preparation of the immediate release pharmaceutical composition includes two methods, a) direct compression method and b) wet granulation method.

In one process, the preparation of immediate release pharmaceutical composition using direct compression method comprises the following steps:
a) weighing the active ingredient and one or two diluent (s) and sieving through sieve number 40;
b) mixing the sieved active ingredient and one or two diluent (s);
c) weighing the lubricant, glidant, disintegrant and acidifying agent and sieving through sieve number 40;
d) adding the mixture obtained in step (c) into step (b) and blending the mixture for 5-20 minutes to form homogenous mixture; and
e) compressing the lubricated blend to obtain the required dosage form.

The above obtained dosage forms can be optionally coated with polymers, solvents and coloring agents by methods known in the art.

In another process, the preparation of immediate release pharmaceutical composition using wet granulation method comprises the following steps:
a) weighing the active ingredient, diluents and super disintegrant;
b) sieving the weighed materials through sieve number 40;
c) blending the sieved active ingredient, diluents and super disintegrant for 10 minutes in an octagonal blender;
d) weighing the binder and dissolve in required quantity of purified water;
e) transferring the active ingredient, diluents and super disintegrant into RMG;
f) adding binder solution dropwise to RMG to form cohesive mass;
g) drying the blend in a tray drier at 50 °C;
h) passing the blend through # 18 mesh to form granules;
i) weighing the lubricant and glidant and pass through sieve number 40;
j) adding the mixture obtained in step (h) to step (i) and blend for 10 minutes in an octagonal blender; and
k) compressing the lubricated blend to obtain the required dosage form.

The above obtained dosage forms can be optionally coated with polymers, solvents and coloring agents by methods known in the art.

### Abbreviations:

- AUC: : Area under the curve
- Cₘₐₓ: : Maximum plasma concentration
- HDPE: : High density polyethylene
- HPMC: : Hydroxypropyl methylcellulose
- HPLC: : High performance liquid chromatography
- kg: : Kilogram
- LC-MS/MS: : Liquid chromatography/ Tandem mass spectrometry
- mg: : Milligram
- mL: : Milliliter
- ng: : Nanogram
- N: : Normality
- rpm: : Rotation per minute
- RMG: : Rapid mixer granulator
- Tₘₐₓ: : Time of maximum plasma concentration
- T_{1/2}: : Half-life
- °C: : Degree Celsius
- % W/W: : Percent weight/weight
- UV: : Ultra violet

### Examples

The following Examples are provided to illustrate preferred embodiments of the invention and are not intended to limit the scope of the present invention.

### Example 1: Pharmaceutical composition of Compound 1 IR tablets.

By using range of ingredients (% w/w) in below mentioned table and procedures explained in above mentioned preparation methods, the IR tablets of 1-[(2-bromophenyl)sulfonyl]-5 -methoxy-3 - [(4-methyl-1 -piperazinyl)methyl] -1H-indole dimesylate monohydrate are prepared.

| **Ingredient** | **Range (% w/w)** |
|---|---|
| Compound 1 | 2 - 60 |
| Binder | 0 - 5 |
| Diluent | 36 - 97 |
| Disintegrant | 0 - 4 |
| Lubricant | 0.5 - 2 |
| Glidant | 0.5 - 1 |
| Acidifying agent | 0 - 2 |

### Example 2:

### Preparation of IR tablet using direct compression method:

### Composition of 5 mg dose IR tablet:

| Ingredient | % w/w | mg/tablet |
|---|---|---|
| Compound 1 | 2.47 | 7.41^{#} |
| Microcrystalline cellulose (Avicel PH 102) | 96.03 | 288.09 |
| Magnesium stearate | 1 | 3 |
| Colloidal silicon dioxide (Aerosil®) | 0.5 | 1.5 |
| Total | 100 | 300 |

| | | |
|---|---|---|
| ^{#} equivalent to 5 mg of 1-[(2-bromophenyl)sulfonyl]-5-methoxy-3-[(4-methyl-1-piperazinyl) methyl]-1H-indole (free base compound). | | |

### Method of preparing IR tablet:

All the ingredients were accurately weighed (Compound 1 of 2.47 %, Avicel PH 102 of 96.03 %) and sieved using sieve number 40. The sieved compound 1 and Avicel PH 102 were blended for 10 minutes in an octagonal blender. The mixture obtained was added to magnesium stearate (1%) and aerosil (0.5 %) and blended for 10 minutes in an octagonal blender. The lubricated blend was compressed using 9 mm round concave punches and dies on rotary compression machine to obtain 300 mg tablet.

The examples 3 to 52 are prepared by following the method of preparation of example 2 by using appropriate amount of active ingredient, diluent(s), disintegrant, lubricant and with/without acidifying agent.

### Examples 3 to 16:

### Compositions of 25 mg dose IR tablets:

| | Example 3 | | Example 4 | |
|---|---|---|---|---|
| Ingredient | (% w/w) | mg/tablet | (% w/w) | mg/tablet |
| Compound 1 | 12.34 | 37.02^{#} | 14.81 | 37.02^{#} |
| Microcrystalline cellulose (Avicel PH 102) | 86.16 | 258.48 | 83.69 | 209.23 |
| Magnesium stearate | 1 | 3 | 1 | 2.5 |
| Colloidal silicon dioxide (Aerosil®) | 0.5 | 1.5 | 0.5 | 1.25 |
| Total | 100 | 300 | 100 | 250 |

| | | | | |
|---|---|---|---|---|
| ^{#} equivalent to 25 mg of 1-[(2-bromophenyl)sulfonyl]-5-methoxy-3-[(4-methyl-1-piperazinyl) methyl]-1H-indole (free base compound) | | | | |

| | Example 5 | | Example 6 | |
|---|---|---|---|---|
| Ingredient | (% w/w) | mg/tablet | (% w/w) | mg/tablet |
| Compound 1 | 37.03 | 37.03^{#} | 12.42 | 37.26^{#} |
| Microcrystalline cellulose (Avicel PH 102) | 61.47 | 61.47 | 84.08 | 252.24 |
| Magnesium stearate | 1 | 1 | 1 | 3 |
| Colloidal silicon dioxide (Aerosil®) | 0.5 | 0.5 | 0.5 | 1.5 |
| Crospovidone | - | - | 2 | 6 |
| Total | 100 | 100 | 100 | 300 |

| | | | | |
|---|---|---|---|---|
| ^{#} equivalent to 25 mg of 1-[(2-bromophenyl)sulfonyl]-5-methoxy-3-[(4-methyl-1-piperazinyl)methyl]-1H-indole (free base compound) | | | | |

| | Example 7 | | Example 8 | |
|---|---|---|---|---|
| Ingredient | (% w/w) | mg/tablet | (% w/w) | mg/tablet |
| Compound 1 | 16.93 | 37.25^{#} | 16.93 | 37.25^{#} |
| Microcrystalline cellulose (Avicel PH 102) | 79.57 | 175.05 | 81.57 | 179.45 |
| Magnesium stearate | 1 | 2.2 | 1 | 2.2 |
| Colloidal silicon dioxide (Aerosil®) | 0.5 | 1.1 | 0.5 | 1.1 |
| Crospovidone | 2 | 4.4 | - | - |
| Total | 100 | 220 | 100 | 220 |

| | | | | |
|---|---|---|---|---|
| equivalent to 25 mg of 1-[(2-bromophenyl)sulfonyl]-5-methoxy-3-[(4-methyl-1-piperazinyl)methyl]-1H-indole (free base compound) | | | | |

| | Example 9 | | Example 10 | |
|---|---|---|---|---|
| Ingredient | (% w/w) | mg/tablet | (% w/w) | mg/tablet |
| Compound 1 | 16.93 | 37.25^{#} | 16.93 | 37.25^{#} |
| Microcrystalline cellulose (Avicel PH 102) | 40.78 | 89.72 | - | - |
| Lactose Monohydrate | 40.79 | 89.73 | - | - |
| Dibasic calcium phosphate dihydrate | - | - | 81.57 | 179.45 |
| Magnesium stearate | 1 | 2.2 | 1 | 2.2 |
| Colloidal silicon dioxide (Aerosil®) | 0.5 | 1.1 | 0.5 | 1.1 |
| Total | 100 | 220 | 100 | 220 |

| | | | | |
|---|---|---|---|---|
| ^{#} equivalent to 25 mg of 1-[(2-bromophenyl)sulfonyl]-5-methoxy-3-[(4-methyl-1-piperazinyl)methyl]-1H-indole (free base compound) | | | | |

| | Example 11 | | Example 12 | |
|---|---|---|---|---|
| Ingredient | (% w/w) | mg/tablet | (% w/w) | mg/tablet |
| Compound 1 | 16.93 | 37.25^{#} | 16.93 | 37.25^{#} |
| Microcrystalline cellulose (Avicel PH 102) | 40.78 | 89.72 | 40.78 | 89.72 |
| Lactose Monohydrate | - | - | 40.79 | 89.73 |
| Starch (Starlac) | 40.79 | 89.73 | - | - |
| Magnesium stearate | 1 | 2.2 | 1 | 2.2 |
| Colloidal silicon dioxide (Aerosil®) | 0.5 | 1.1 | 0.5 | 1.1 |
| Total | 100 | 220 | 100 | 220 |

| | | | | |
|---|---|---|---|---|
| ^{#} equivalent to 25 mg of 1-[(2-bromophenyl)sulfonyl]-5-methoxy-3-[(4-methyl-1 piperazinyl)methyl]-1H-indole (free base compound) | | | | |

| | Example 13 | | Example 14 | |
|---|---|---|---|---|
| Ingredient | (% w/w) | mg/tablet | (% w/w) | mg/tablet |
| Compound 1 | 16.93 | 37.25^{#} | 16.93 | 37.25^{#} |
| Microcrystalline cellulose (Avicel PH 102) | 40.78 | 89.72 | 81.57 | 179.45 |
| Starch (Starlac) | 40.79 | 89.73 | - | - |
| Magnesium stearate | 1 | 2.2 | 1 | 2.2 |
| Colloidal silicon dioxide (Aerosil®) | 0.5 | 1.1 | 0.5 | 1.1 |
| Total | 100 | 220 | 100 | 220 |

| | | | | |
|---|---|---|---|---|
| ^{#} equivalent to 25 mg of 1-[(2-bromophenyl)sulfonyl]-5-methoxy-3-[(4-methyl-1-piperazinyl)methyl]-1H-indole (free base compound) | | | | |

| | Example 15 | | Example 16 | |
|---|---|---|---|---|
| Ingredient | (% w/w) | mg/tablet | (% w/w) | mg/tablet |
| Compound 1 | 16.93 | 37.25^{#} | 16.93 | 37.25^{#} |
| Microcrystalline cellulose (Avicel PH 102) | - | - | 80.57 | 177.25 |
| Starch (Starlac) | 81.57 | 179.45 | - | - |
| Magnesium stearate | 1 | 2.2 | 1 | 2.2 |
| Colloidal silicon dioxide (Aerosil®) | 0.5 | 1.1 | 0.5 | 1.1 |
| Citric acid | - | - | 1 | 2.2 |
| Total | 100 | 220 | 100 | 220 |

| | | | | |
|---|---|---|---|---|
| ^{#} equivalent to 25 mg of 1-[(2-bromophenyl)sulfonyl]-5-methoxy-3-[(4-methyl-1-piperazinyl)methyl]-1H-indole (free base compound) | | | | |

### Examples 17 to 38:

### Compositions of 50 mg dose IR tablets:

| | Example 17 | | Example 18 | |
|---|---|---|---|---|
| Ingredient | (% w/w) | mg/tablet | (% w/w) | mg/tablet |
| Compound 1 | 29.62 | 74.05^{#} | 29.62 | 74.05^{#} |
| Microcrystalline cellulose (Avicel PH 102) | 66.63 | 166.58 | - | - |
| Isomalt | - | - | 68.88 | 172.2 |
| Magnesium stearate | 1.25 | 3.12 | 1 | 2.5 |
| Colloidal silicon dioxide (Aerosil®) | 0.5 | 1.25 | 0.5 | 1.25 |
| Crospovidone | 2 | 5 | - | - |
| Total | 100 | 250 | 100 | 250 |

| | | | | |
|---|---|---|---|---|
| ^{#} equivalent to 50 mg of 1-[(2-bromophenyl)sulfonyl]-5-methoxy-3-[(4-methyl-1-piperazinyl)methyl]-1H-indole (free base compound) | | | | |

| | Example 19 | | Example 20 | |
|---|---|---|---|---|
| Ingredient | (% w/w) | mg/tablet | (% w/w) | mg/tablet |
| Compound 1 | 29.62 | 74.05^{#} | 28.8 | 72^{#} |
| Starch (Starlac) | 68.88 | 172.2 | - | - |
| Microcrystalline cellulose (Avicel PH 113) | - | - | 69.45 | 173.63 |
| Magnesium stearate | 1 | 2.5 | 1.25 | 3.12 |
| Colloidal silicon dioxide (Aerosil®) | 0.5 | 1.25 | 0.5 | 1.25 |
| Total | 100 | 250 | 100 | 250 |

| | | | | |
|---|---|---|---|---|
| ^{#} equivalent to 50 mg of 1-[(2-bromophenyl)sulfonyl]-5-methoxy-3-[(4-methyl-1-piperazinyl)methyl]-1H-indole (free base compound) | | | | |

| | Example 21 | | Example 22 | |
|---|---|---|---|---|
| Ingredient | (% w/w) | mg/tablct | (% w/w) | mg/tablct |
| Compound 1 | 28.8 | 72^{#} | 29.62 | 74.05^{#} |
| Microcrystalline cellulose (Avicel PH 102) | 69.45 | 173.63 | - | - |
| Lactose Monohydrate | - | - | 68.63 | 171.58 |
| Magnesium stearate | 1.25 | 3.12 | 1.25 | 3.12 |
| Colloidal silicon dioxide (Aerosil®) | 0.5 | 1.25 | 0.5 | 1.25 |
| Total | 100 | 250 | 100 | 250 |

| | | | | |
|---|---|---|---|---|
| ^{#} equivalent to 50 mg of 1-[(2-bromophenyl)sulfonyl]-5-methoxy-3-[(4-methyl-1-piperazinyl)methyl]-1H-indole (free base compound) | | | | |

| | Example 23 | | Example 24 | |
|---|---|---|---|---|
| Ingredient | (% w/w) | mg/tablet | (% w/w) | mg/tablet |
| Compound 1 | 29.62 | 74.05^{#} | 28.8 | 72^{#} |
| Starch (Starcap 1500) | 68.63 | 171.58 | - | - |
| Microcrystalline cellulose (Avicel PH 101) | - | - | 69.45 | 173.63 |
| Magnesium stearate | 1.25 | 3.12 | 1.25 | 3.12 |
| Colloidal silicon dioxide (Aerosil®) | 0.5 | 1.25 | 0.5 | 1.25 |
| Total | 100 | 250 | 100 | 250 |

| | | | | |
|---|---|---|---|---|
| ^{#} equivalent to 50 mg of 1-[(2-bromophenyl)sulfonyl]-5-methoxy-3-[(4-methyl-1-piperazinyl)methyl]-1H-indole (free base compound) | | | | |

| | Example 25 | | Example 26 | |
|---|---|---|---|---|
| Ingredient | (% w/w) | mg/tablet | (% w/w) | mg/tablet |
| Compound 1 | 29.62 | 74.05^{#} | 29.62 | 74.05^{#} |
| Dextrose Monohydrate | 68.88 | 172.2 | - | - |
| Mannitol | - | - | 68.88 | 172.2 |
| Magnesium stearate | 1 | 2.5 | 1 | 2.5 |
| Colloidal silicon dioxide (Aerosil®) | 0.5 | 1.25 | 0.5 | 1.25 |
| Total | 100 | 250 | 100 | 250 |

| | | | | |
|---|---|---|---|---|
| ^{#} equivalent to 50 mg of 1-[(2-bromophenyl)sulfonyl]-5-methoxy-3-[(4-methyl-1-piperazinyl)methyl]-1H-indole (free base compound) | | | | |

| | Example 27 | | Example 28 | |
|---|---|---|---|---|
| Ingredient | (% w/w) | mg/tablet | (% w/w) | mg/tablet |
| Compound 1 | 37.03 | 74.06^{#} | 29.62 | 74.05^{#} |
| Microcrystalline cellulose (Avicel PH 102) | 61.47 | 122.94 | - | - |
| Dicalcium phosphate dihydrate | - | - | 68.63 | 171.58 |
| Magnesium stearate | 1 | 2 | 1.25 | 3.12 |
| Colloidal silicon dioxide (Aerosil®) | 0.5 | 1 | 0.5 | 1.25 |
| Total | 100 | 200 | 100 | 250 |

| | | | | |
|---|---|---|---|---|
| ^{#} equivalent to 50 mg of 1-[(2-bromophenyl)sulfonyl]-5-methoxy-3-[(4-methyl-1-piperazinyl)methyl]-1H-indole (free base compound) | | | | |

| | Example 29 | | Example 30 | |
|---|---|---|---|---|
| Ingredient | (% w/w) | mg/tablet | (% w/w) | mg/tablet |
| Compound 1 | 29.62 | 74.05^{#} | 29.62 | 74.05^{#} |
| Microcrystalline cellulose (Avicel PH 101) | 68.63 | 171.58 | - | - |
| Lactose Monohydrate | - | - | 66.63 | 166.58 |
| Magnesium stearate | 1.25 | 3.12 | 1.25 | 3.12 |
| Colloidal silicon dioxide (Aerosil®) | 0.5 | 1.25 | 0.5 | 1.25 |
| Crospovidone | - | - | 2 | 5 |
| Total | 100 | 250 | 100 | 250 |

| | | | | |
|---|---|---|---|---|
| equivalent to 50 mg of 1-[(2-bromophenyl)sulfonyl]-5-methoxy-3-[(4-methyl-1-piperazinyl)methyl]-1H-indole (free base compound) | | | | |

| | Example 31 | | Example 32 | |
|---|---|---|---|---|
| Ingredient | (% w/w) | mg/tablet | (% w/w) | mg/tablet |
| Compound 1 | 29.62 | 74.05^{#} | 29.62 | 74.05^{#} |
| Starch (Starcap 1500) | - | - | 66.63 | 166.58 |
| Dicalcium phosphate dihydrate | 66.63 | 166.58 | - | - |
| Magnesium stearate | 1.25 | 3.12 | 1.25 | 3.12 |
| Colloidal silicon dioxide (Aerosil®) | 0.5 | 1.25 | 0.5 | 1.25 |
| Crospovidone | 2 | 5 | 2 | 5 |
| Total | 100 | 250 | 100 | 250 |

| | | | | |
|---|---|---|---|---|
| # equivalent to 50 mg of 1-[(2-bromophenyl)sulfonyl]-5-methoxy-3-[(4-methyl-1-piperazinyl)methyl]-1H-indole (free base compound) | | | | |

| | Example 33 | | Example 34 | |
|---|---|---|---|---|
| Ingredient | (% w/w) | mg/tablet | (% w/w) | mg/tablet |
| Compound 1 | 29.62 | 74.05^{#} | 29.62 | 74.05^{#} |
| Lactose Monohydrate | 66.63 | 166.58 | - | - |
| Microcrystalline cellulose (Avicel 101) | - | - | 66.63 | 166.58 |
| Magnesium stearate | 1.25 | 3.12 | 1.25 | 3.12 |
| Colloidal silicon dioxide (Aerosil®) | 0.5 | 1.25 | 0.5 | 1.25 |
| Crospovidone | 2 | 5 | 2 | 5 |
| Total | 100 | 250 | 100 | 250 |

| | | | | |
|---|---|---|---|---|
| # equivalent to 50 mg of 1-[(2-bromophenyl)sulfonyl]-5-methoxy-3-[(4-methyl-1-piperazinyl)methyl]-1H-indole (free base compound) | | | | |

| | Example 35 | | Example 36 | |
|---|---|---|---|---|
| Ingredient | (% w/w) | mg/tablet | (% w/w) | mg/tablet |
| Compound 1 | 24.83 | 74.5^{#} | 33.86 | 74.5^{#} |
| Microcrystalline cellulose (Avicel PH 102) | 71.67 | 215 | 62.64 | 137.8 |
| Magnesium stearate | 1 | 3 | 1 | 2.2 |
| Colloidal silicon dioxide (Aerosil®) | 0.5 | 1.5 | 0.5 | 1.1 |
| Crospovidone | 2 | 6 | 2 | 4.4 |
| Total | 100 | 300 | 100 | 220 |

| | | | | |
|---|---|---|---|---|
| # equivalent to 50 mg of 1-[(2-bromophenyl)sulfonyl]-5-methoxy-3-[(4-methyl-1-piperazinyl)methyl]-1H-indole (free base compound) | | | | |

| | Example 37 | | Example 38 | |
|---|---|---|---|---|
| Ingredient | (% w/w) | mg/tablet | (% w/w) | mg/tablet |
| Compound 1 | 12.35 | 74.1^{#} | 49.67 | 74.51^{#} |
| Microcrystalline cellulose (Avicel PH 102) | 84.15 | 504.9 | 49.83 | 70.24 |
| Magnesium stearate | 1 | 6 | 1 | 1.5 |
| Colloidal silicon dioxide (Aerosil®) | 0.5 | 3 | 0.5 | 0.75 |
| Crospovidone | 2 | 12 | 2 | 3 |
| Total | 100 | 600 | 100 | 150 |

| | | | | |
|---|---|---|---|---|
| ^{#} equivalent to 50 mg of 1-[(2-bromophenyl)sulfonyl]-5-methoxy-3-[(4-methyl-1-piperazinyl)methyl]-1H-indole (free base compound) | | | | |

### Example 39 to 43:

### Composition of 75 mg dose IR tablets:

| | Example 39 | | Example 40 | |
|---|---|---|---|---|
| Ingredient | (% w/w) | mg/tablet | (% w/w) | mg/tablet |
| Compound 1 | 37.25 | 111.75^{#} | 50.79 | 111.74^{#} |
| Microcrystalline cellulose (Avicel PH 102) | 59.25 | 177.75 | 45.71 | 100.56 |
| Magnesium stearate | 1 | 3 | 1 | 2.2 |
| Colloidal silicon dioxide (Aerosil®) | 0.5 | 1.5 | 0.5 | 1.1 |
| Crospovidone | 2 | 6 | 2 | 4.4 |
| Total | 100 | 300 | 100 | 220 |

| | | | | |
|---|---|---|---|---|
| ^{#} equivalent to 75 mg of 1-[(2-bromophenyl)sulfonyl]-5-methoxy-3-[(4-methyl-1-piperazinyl)methyl]-1H-indole (free base compound) | | | | |

| | Example 41 | | Example 42 | |
|---|---|---|---|---|
| Ingredient | (% w/w) | mg/tablet | (% w/w) | mg/tablet |
| Compound 1 | 50.8 | 111.76^{#} | 50.8 | 111.76^{#} |
| Microcrystalline cellulose (Avicel PH 102) | 46.7 | 102.74 | 47.7 | 104.94 |
| Magnesium stearate | 1 | 2.2 | 1 | 2.2 |
| Colloidal silicon dioxide (Aerosil®) | 0.5 | 1.1 | 0.5 | 1.1 |
| Citric acid | 1 | 2.2 | - | - |
| Total | 100 | 220 | 100 | 220 |

| | | | | |
|---|---|---|---|---|
| equivalent to 75 mg of 1-[(2-bromophenyl)sulfonyl]-5-methoxy-3-[(4-methyl-1-piperazinyl)methyl]-1H-indole (free base compound) | | | | |

| | Example 43 | |
|---|---|---|
| Ingredient | (% w/w) | mg/tablet |
| Compound 1 | 50.8 | 111.76^{#} |
| Starch (Starlac) | 47.7 | 104.98 |
| Magnesium stearate | 1 | 2.2 |
| Colloidal silicon dioxide (Aerosil®) | 0.5 | 1.1 |
| Total | 100 | 220 |

| | | |
|---|---|---|
| ^{#} equivalent to 75 mg of 1-[(2-bromophenyl)sulfonyl]-5-methoxy-3-[(4-methyl-1-piperazinyl)methyl]-1H-indole (free base compound) | | |

### Examples 44 to 50:

### Composition of 100 mg dose IR tablets:

| | Example 44 | | Example 45 | |
|---|---|---|---|---|
| Ingredient | (% w/w) | mg/tablet | (% w/w) | mg/tablet |
| Compound 1 | 49.36 | 148.08^{#} | 49.37 | 148.11^{#} |
| Microcrystalline cellulose (Avicel PH 102) | 49.14 | 147.42 | 49.13 | 147.39 |
| Magnesium stearate | 1 | 3 | 1 | 3 |
| Colloidal silicon dioxide (Aerosil®) | 0.5 | 1.5 | 0.5 | 1.5 |
| Total | 100 | 300 | 100 | 300 |

| | | | | |
|---|---|---|---|---|
| equivalent to 100 mg of 1-[(2-bromophenyl)sulfonyl]-5-methoxy-3-[(4-methyl-1-piperazinyl)methyl]-1H-indole (free base compound) | | | | |

| | Example 46 | | Example 47 | |
|---|---|---|---|---|
| Ingredient | (% w/w) | mg/tablet | (% w/w) | mg/tablet |
| Compound 1 | 59.24 | 148.1^{#} | 37.03 | 148.12^{#} |
| Microcrystalline cellulose (Avicel PH 102) | 39.26 | 98.15 | 61.47 | 245.88 |
| Magnesium stearate | 1 | 2.5 | 1 | 4 |
| Colloidal silicon dioxide | 0.5 | 1.25 | 0.5 | 2 |
| (Aerosil®) | | | | |
| Total | 100 | 250 | 100 | 400 |

| | | | | |
|---|---|---|---|---|
| ^{#} equivalent to 100 mg of 1-[(2-bromophenyl)sulfonyl]-5-methoxy-3-[(4-methyl-1-piperazinyl)methyl]-1H-indole (free base compound) | | | | |

| | Example 48 | | Example 49 | |
|---|---|---|---|---|
| Ingredient | (% w/w) | mg/tablet | (% w/w) | mg/tablet |
| Compound 1 | 49.67 | 149.01^{#} | 59.6 | 149^{#} |
| Microcrystalline cellulose (Avicel PH 102) | 47.33 | 141.99 | 36.9 | 92.25 |
| Magnesium stearate | 0.5 | 1.5 | 1 | 2.5 |
| Colloidal silicon dioxide (Aerosil®) | 0.5 | 1.5 | 0.5 | 1.25 |
| Crospovidone | 2 | 6 | 2 | 5 |
| Total | 100 | 300 | 100 | 250 |

| | | | | |
|---|---|---|---|---|
| equivalent to 100 mg of 1-[(2-bromophenyl)sulfonyl]-5-methoxy-3-[(4-methyl-1-piperazinyl)methyl]-1H-indole (free base compound) | | | | |

| | Example 50 | |
|---|---|---|
| Ingredient | (% w/w) | mg/tablet |
| Compound 1 | 24.7 | 148.2^{#} |
| Microcrystalline cellulose (Avicel PH 102) | 71.8 | 430.8 |
| Magnesium stearate | 1 | 6 |
| Colloidal silicon dioxide (Aerosil®) | 0.5 | 3 |
| Crospovidone | 2 | 12 |
| Total | 100 | 600 |

| | | |
|---|---|---|
| ^{#} equivalent to 100 mg of 1-[(2-bromophenyl)sulfonyl]-5-methoxy-3-[(4-methyl-1-piperazinyl)methyl]-1H-indole (free base compound) | | |

### Examples 51-52:

### Composition of 150 mg and 200 mg dose IR tablets:

| | Example 51 | | Example 52 | |
|---|---|---|---|---|
| Ingredient | (% w/w) | mg/tablet | (% w/w) | mg/tablet |
| Compound 1 | 49.67 | 223.52* | 49.67 | 298.02^{#} |
| Microcrystalline cellulose (Avicel PH 102) | 46.83 | 210.73 | 46.83 | 280.98 |
| Magnesium stearate | 1 | 4.5 | 1 | 6 |
| Colloidal silicon dioxide (Aerosil®) | 0.5 | 2.25 | 0.5 | 3 |
| Crospovidone | 2 | 9 | 2 | 12 |
| Total | 100 | 450 | 100 | 600 |

| | | | | |
|---|---|---|---|---|
| ^{*} equivalent to 150 mg of 1-[(2-bromophenyl)sulfonyl]-5-methoxy-3-[(4-methyl-1-piperazinyl)methyl]-1H-indole (free base compound) ^{#} equivalent to 200 mg of 1-[(2-bromophenyl)sulfonyl]-5-methoxy-3-[(4-methyl-1-piperazinyl)methyl]-1H-indole (free base compound) | | | | |

### Example 53:

### Preparation of IR tablet using wet granulation method

### Composition of 50 mg IR tablet:

| Ingredient | % W/W | mg/tablet |
|---|---|---|
| Compound 1 | 24.67 | 74^{#} |
| Microcrystalline cellulose (Avicel PH 102) | 66.83 | 200.5 |
| Povidone | 4.0 | 12 |
| Crospovidone | 3.0 | 9 |
| Magnesium stearate | 1.0 | 3 |
| Colloidal silicon dioxide (Aerosil®) | 0.5 | 1.5 |
| Total | 100 | 300 |

| | | |
|---|---|---|
| ^{#} equivalent to 50 mg of 1-[(2-bromophenyl)sulfonyl]-5-methoxy-3-[(4-methyl-1-piperazinyl)methyl]-1H-indole (free base compound) | | |

### Method of preparing IR tablet:

All the ingredients were accurately weighed (Compound 1 of 24.67%, Avicel PH 102 of 66.83% and crospovidone of 3%) and sieved using sieve number 40. The sieved compound 1, Avicel PH 102 and crospovidone were blended for 10 minutes in an octagonal blender. The mixture obtained was transferred into RMG and added povidone binder solution (povidone (4 %) was dissolved in purified water) dropwise to RMG to form cohesive mass. The blend obtained was dried in a tray drier at 50 °C. Dried blend was passed through # 18 mesh to form granules. The granules obtained were mixed with magnesium stearate and aerosil and the mixture was blended for 10 minutes in an octagonal blender. The lubricated blend was compressed using 9 mm round concave punches and dies on rotary compression machine to obtain 300 mg tablet.

### Examples 54-55:

The following examples are prepared by following the method of preparation of example 53.

### Composition of 50 mg IR tablets:

| | Example 54 | | Example 55 | |
|---|---|---|---|---|
| Ingredient | (% w/w) | mg/tablet | (% w/w) | mg/tablet |
| Compound 1 | 24.67 | 74^{#} | 24.67 | 74^{#} |
| Microcrystalline cellulose (Avicel PH 102) | 65.83 | 197.5 | 64.83 | 194.5 |
| Povidone (PVP K30) | 4.0 | 12 | - | - |
| HPMC | - | - | 5.0 | 15 |
| Sodium starch glycolate | 4.0 | 12 | - | - |
| Croscarmellose sodium | - | - | 4.0 | 12 |
| Magnesium stearate | 1 | 3 | 1 | 3 |
| Aerosil | 0.5 | 1.5 | 0.5 | 1.5 |
| Total | 100 | 300 | 100 | 300 |

| | | | | |
|---|---|---|---|---|
| ^{#} equivalent to 50 mg of 1-[(2-bromophenyl)sulfonyl]-5-methoxy-3-[(4-methyl-1-piperazinyl)methyl]-1H-indole (free Base compound) | | | | |

### Example 56:

### Dissolution studies of IR tablets

The dissolution studies were conducted for the immediate release tablets of the instant invention to demonstrate the % release of active ingredient at different time intervals. Protocol:
Dissolution was carried out in accordance with the United States pharmacopeia general procedures using dissolution apparatus II (paddle method). The IR tablet was placed in 900 mL of simulated gastric fluid (pH 1.2), 0.1N hydrochloric acid or water at 37 °C with a paddle speed of 50 rpm/ 100 rpm and measuring the amount of active ingredient dissolved (especially, using UV at 255 nm or using HPLC, wavelength 220 nm) at 15 and 30 minutes. Results:
The dissolution studies data of the IR tablets are tabulated below.

| S. No | Examples | Time (minutes) | % of Active ingredient release | S. No | Examples | Time (minutes) | % of Active ingredient release |
|---|---|---|---|---|---|---|---|
| 1 | Example 2 | 15 | 97 | 19 | Example 25 | 15 | 98 |
| | | 30 | 97 | | | 30 | 97 |
| 2 | Example 3 | 15 | 108 | 20 | Example 26 | 15 | 104 |
| | | 30 | 105 | | | 30 | 103 |
| 3 | Example 4 | 15 | 85 | 21 | Example 27 | 15 | 102 |
| | | 30 | 97 | | | 30 | 106 |
| 4 | Example 6 | 15 | 103 | 22 | Example 30 | 15 | 104 |
| | | 30 | 106 | | | 30 | 104 |
| 5 | Example 7 | 15 | 103 | 23 | Example 32 | 15 | 98 |
| | | 30 | 103 | | | 30 | 100 |
| 6 | Example 12 | 15 | 103 | 24 | Example 33 | 15 | 102 |
| | | 30 | 104 | | | 30 | 101 |
| 7 | Example 13 | 15 | 97 | 25 | Example 37 | 15 | 98 |
| | | | | | | 30 | 96 |
| | | 30 | 100 | 26 | Example 38 | 15 | 98 |
| | | | | | | 30 | 102 |
| 8 | Example 14 | 15 | 100 | 27 | Example 40 | 15 | 101 |
| | | 30 | 100 | | | 30 | 101 |
| 9 | Example 15 | 15 | 102 | 28 | Example 41 | 15 | 100 |
| | | 30 | 102 | | | 30 | 100 |
| 10 | Example 16 | 15 | 96 | 29 | Example 42 | 15 | 100 |
| | | 30 | 97 | | | 30 | 100 |
| 11 | Example 17 | 15 | 102 | 30 | Example 43 | 15 | 106 |
| | | 30 | 101 | | | 30 | 108 |
| 12 | Example 18 | 15 | 98 | 31 | Example 47 | 15 | 82 |
| | | 30 | 98 | | | 30 | 94 |
| 13 | Example 19 | 15 | 100 | 32 | Example 48 | 15 | 105 |
| | | 30 | 99 | | | 30 | 105 |
| 14 | Example 20 | 15 | 102 | 33 | Example 50 | 15 | 101 |
| | | | | | | 30 | 99 |
| | | 30 | 107 | 34 | Example 51 | 15 | 98 |
| | | | | | | 30 | 96 |
| 15 | Example 21 | 15 | 107 | 35 | Example 52 | 15 | 99 |
| | | | | | | 30 | 99 |
| | | 30 | 106 | 33 | Example 53 | 15 | 99 |
| 16 | Example 22 | 15 | 101 | | | 30 | 99 |
| | | 30 | 103 | 34 | Example 54 | 15 | 100 |
| 17 | Example 23 | 15 | 99 | | | 30 | 100 |
| | | 30 | 99 | 35 | Example 55 | 15 | 101 |
| 18 | Example 24 | 15 | 105 | | | 30 | 101 |
| | | 30 | 105 | | | | |

### Example 57:

### Stability study of IR tablets

The stability study was conducted to assess the stability of the immediate release tablets and the impurity profile of the instant invention under different storage conditions.

The stability studies were carried out at ambient temperature, 40 °C / 75 % RH and 60 °C oven for 6 months.

### Protocol:

The immediate release tablets are packed in HDPE bottles with polyethylene liners with desiccant for a period of 6 months at different storage conditions. The samples were analyzed for purity using HPLC.

### Results:

### Dissolution:

The dissolution data of examples for different time points at accelerated storage conditions are tabulated below.

| S. No | Examples | Time (minutes) | % of Active ingredient release | | | |
|---|---|---|---|---|---|---|
| | | | Day 1 | 1 month | 3 months | 6 months |
| 1 | Example 18 | 30 | 106 | 106 | 90 | 104 |
| 2 | Example 20 | 30 | 91 | 99 | 89 | 97 |
| 3 | Example 21 | 30 | 98 | 99 | 103 | 102 |
| 4 | Example 22 | 30 | 99 | 100 | 103 | 99 |
| 5 | Example 37 | 30 | 96 | 99 | 100 | 102 |
| 6 | Example 38 | 30 | 102 | 101 | 100 | 99 |
| 7 | Example 50 | 30 | 99 | 99 | 99 | 99 |

### Conclusion:

We observed no significant variation in dissolution of the IR tablets after storing for 6 months at accelerated storage conditions (*i.e,* temperature 40 ± 2 °C at 75 ± 5 % relative humidity (RH)).

### Purity:

The purity of IR tablet on day 1 is tabulated below.

| S. No | Example number | Dose (mg) | Purity of active ingredient (%) | Chloro impurity (%) | Maximum unknown impurity (%) | Other unknown impurities (%) | Total impurities (%) |
|---|---|---|---|---|---|---|---|
| 1 | 18 | 75 | 99.64 | 0.19 | 0.06 | 0.11 | 0.36 |
| 2 | 20 | 75 | 99.66 | 0.19 | 0.06 | 0.09 | 0.34 |
| 3 | 22 | 75 | 99.64 | 0.20 | 0.06 | 0.10 | 0.36 |

The purity of IR tablets under different storage conditions at the end of 6 months is tabulated below.

| S. No | Example number | Dose (mg) | Storage conditions | Purity (%) | Chloro impurity (%) | Maximum unknown impurity (%) | Other impurities (%) | Total impurities (%) |
|---|---|---|---|---|---|---|---|---|
| 1 | 18 | 75 | 60 °C Oven | 99.38 | 0.21 | 0.09 | 0.32 | 0.62 |
| 2 | 18 | 75 | 40 °C / 75 % RH | 99.63 | 0.19 | 0.06 | 0.12 | 0.37 |
| 3 | 20 | 75 | 40 °C / 75 % RH | 99.64 | 0.19 | 0.06 | 0.11 | 0.36 |
| 4 | 22 | 75 | 60 °C Oven | 99.37 | 0.20 | 0.08 | 0.35 | 0.63 |
| 5 | 22 | 75 | 40 °C / 75 % RH | 99.62 | 0.20 | 0.06 | 0.12 | 0.38 |

### Conclusion:

We observed no significant variation in purity of the active ingredient under different storage conditions. As evident from the above stability data the active ingredient in immediate release tablets of instant invention is stable at least six months under accelerated storage condition.

### Example 58:

### In-vivo pharmacokinetic study of IR tablets

The dog pharmacokinetic study is conducted to confirm the dissolution data of Compound 1.

### Experimental procedure of dog pharmacokinetic study

Male beagle dogs (10 ± 2 kg) were used as experimental animals. Each dog was housed in individual cages. Animals were fasted over night before oral dosing (p.o) and food pellets were allowed 2 hours post dosing. Two beagle dogs (∼11 mg/kg) were dosed orally with IR tablets prepared by pharmaceutical compositions disclosed in Example 48.

At each time point, blood (0.5 mL) was collected through cephalic vein. Collected blood was transferred into a labeled eppendroff tube containing 10 µL of heparin as anticoagulant. Typically blood samples were collected at following time points: Pre dose, 0.25, 0.5, 1, 1.5, 2, 3, 5, 7, 12, 24, 30 and 48 hours post dose (n=2). Blood was centrifuged at 4000 rpm for 10 minutes. Plasma was separated and stored at -20 °C until analysis. The concentrations of active ingredient were quantified in plasma by validated LC-MS/MS method using suitable extraction technique. The active ingredient was quantified in the calibration range around 0.2-200 ng/mL.

Pharmacokinetic parameters Cₘₐₓ, Tₘₐₓ, AUC₀₋ₜ and T_{1/2} were calculated by using standard non-compartmental model Phoenix WinNonlin 6.2 version Software package.

The results of this study are tabulated below.

| Strain/ Gender | Dose (mg/kg) | Dosage form | Cₘₐₓ (ng/mL) | Tₘₐₓ (hours) | AUC₀₋ₜ (ng.hour/mL) | T_{1/2} (hours) |
|---|---|---|---|---|---|---|
| Beagle dog | ∼11 | Tablet | 60 ± 16 | 1.25±0.35 | 251 ± 27 | 5.97±0.40 |

## Claims

1. An immediate release pharmaceutical composition on a total of 100% by weight comprises:
a) from 2 % to 60 % 1-[(2-bromophenyl)sulfonyl]-5-methoxy-3-[(4-methyl-1-piperazinyl)methyl]-1H-indole or a pharmaceutically acceptable salt thereof;
b) from 36 % to 97 % diluent or total of two diluents; wherein the diluent is selected from the group consisting of microcrystalline cellulose, lactose monohydrate, dibasic calcium phosphate, lactose, lactose hydrate, lactose anhydrate, mannitol, starch and isomalt;
c) from 0.5 % to 2 % lubricant; wherein the lubricant is magnesium stearate;
d) from 0.5 % to 1 % glidant; wherein the glidant is colloidal silicon dioxide; e) 0 % to 10 % binder; wherein the binder is selected from group consisting of povidone or hydroxypropyl methylcellulose;
f) 0 % to 5 % disintegrant; wherein the disintegrant is selected from crospovidone, sodium starch glycolate and croscarmellose sodium; and
g) 0 % to 2 % acidifying agent; wherein the acidifying agent is citric acid.

2. The immediate release pharmaceutical composition as claimed in claim 1, wherein said composition comprises on a total of 100 % by weight:
(a) from 2 % to 60 % 1-[(2-bromophenyl)sulfonyl]-5-methoxy-3-[(4-methyl-1-piperazinyl)methyl]-1H-indole dimesylate monohydrate;
(b) from 36 % to 97 % of one diluent or total of two diluents; where in the diluent is selected from the group consisting of microcrystalline cellulose, lactose monohydrate, dibasic calcium phosphate, lactose, lactose hydrate, lactose anhydrate, mannitol, starch and isomalt;
(c) from 0.5 % to 2 % lubricant; wherein the lubricant is magnesium stearate;
(d) from 0.5 % to 1 % glidant; wherein the glidant is colloidal silicon dioxide;
(e) 0 % to 10 % binder; where in the binder is selected from group consisting of povidone or hydroxypropyl methylcellulose;
(f) 0 % to 5 % disintegrant; wherein the disintegrant is selected from crospovidone, sodium starch glycolate and croscarmellose sodium and
(g) 0 % to 2 % acidifying agent; wherein the acidifying agent is citric acid.

3. The immediate release pharmaceutical compositions as claimed in claim 1 or claim 2, wherein the composition on a total of 100 % by weight is selected from the group consisting of:
1) (a) from 2 % to 3 % 1-[(2-bromophenyl)sulfonyl]-5-methoxy-3-[(4-methyl-1-piperazinyl) methyl]-1H-indole dimesylate monohydrate, (b) from 95 % to 97 % diluent, (c) 1 % lubricant and (d) 0.5 % glidant;
2) (a) from 11 % to 38 % 1-[(2-bromophenyl)sulfonyl]-5-methoxy-3-[(4-methyl-1-piperazinyl)methyl]-1H-indole dimesylate monohydrate, (b) from 61 % to 87 % of one diluent or total of two diluents, (c) 1% lubricant, (d) 0.5 % glidant, (e) 2 % disintegrant and (f) 1 % acidifying agent;
3) (a) from 24 % to 38 % 1-[(2-bromophenyl)sulfonyl]-5-methoxy-3-[(4-methyl-1-piperazinyl)methyl]-1H-indole dimesylate monohydrate (b) from 61 % to 72 % one diluent or total of two diluents, (c) from 1 % to 1.25 % lubricant, (d) 0.5 % glidant and (e) 2 % disintegrant;
4) (a) from 37 % to 51 % 1-[(2-bromophenyl)sulfonyl]-5-methoxy-3-[(4-methyl-1-piperazinyl)methyl]-1H-indole dimesylate monohydrate, (b) from 45 % to 60 % diluent, (c) 1 % lubricant, (d) 0.5 % glidant, (e) 2 % of disintegrant and (f) 1 % acidifying agent;
5) (a) from 36 % to 60 % 1-[(2-bromophenyl)sulfonyl]-5-methoxy-3-[(4-methyl-1-piperazinyl)methyl]-1H-indole dimesylate monohydrate, (b) from 36 % to 62 % diluent, (c) from 0.5 % to 1 % lubricant, (d) 0.5 % glidant and (e) 2 % disintegrant; and
6) (a) from 11 % to 38 % 1-[(2-bromophenyl)sulfonyl]-5-methoxy-3-[(4-methyl-1-piperazinyl)methyl]-1H-indole dimesylate monohydrate; (b) from 61 % to 72 % diluent; (c) from 2 % to 5 % binder; (d) 1 % lubricant; (e) 0.5 % glidant and (f) from 2 % to 4 % disintegrant.

4. The immediate release pharmaceutical composition as claimed in any one of the claims 1 to 3, wherein the dosage of 1-[(2-bromophenyl)sulfonyl]-5-methoxy-3-[(4-methyl-1-piperazinyl)methyl]-1H-indole or a pharmaceutically acceptable salt thereof is from 5 mg to 200 mg.

5. The immediate release pharmaceutical composition as claimed in any one of the claims 1 to 4, wherein the composition is in the form of tablet or capsule.

6. The immediate release pharmaceutical composition as claimed in any one of the claims 1 to 3, wherein the composition having:
i) less than 0.5 % of chloro impurity;
ii) less than 0.5 % of unknown impurity;
iii) less than 1 % of total impurity.

7. The immediate release pharmaceutical composition as claimed in any one of the claims 1 to 3, wherein the purity of 1-[(2-bromophenyl)sulfonyl]-5-methoxy-3-[(4-methyl-1-piperazinyl)methyl]-1H-indole dimesylate monohydrate is 99.3 %.

8. The immediate release pharmaceutical composition as claimed in any one of the claims 1 to 3, wherein the composition having:
i) 99.3 % purity of 1-[(2-bromophenyl)sulfonyl]-5-methoxy-3-[(4-methyl-1-piperazinyl)methyl]-1H-indole dimesylate monohydrate;
ii) less than 0.5 % of chloro impurity;
iii) less than 0.5 % of unknown impurity;
iv) less than 1 % of total impurity.

9. The immediate release pharmaceutical composition as claimed in any one of the claims 1 to 3, wherein the 1-[(2-bromophenyl)sulfonyl]-5-methoxy-3-[(4-methyl-1-piperazinyl) methyl]-1H-indole dimesylate monohydrate is released from 85 % to 100 % within 30 minutes when tested with the rotating paddle at 100 rpm with 900 mL of dissolution media, 01N hydrochloric acid or water at 37°C.

10. The immediate release pharmaceutical composition as claimed in claim 5, wherein said composition on a total of 100% by weight comprises:
(a) from 2 % to 60 % 1-[(2-bromophenyl)sulfonyl]-5-methoxy-3-[(4-methyl-1-piperazinyl)methyl]-1H-indole dimesylate monohydrate;
(b) from 36 % to 97 % of one diluent or total of two diluents; where in the diluent is selected from the group consisting of microcrystalline cellulose, lactose monohydrate, dibasic calcium phosphate, lactose, lactose hydrate, lactose anhydrate, mannitol, starch and isomalt;
(c) from 0.5 % to 2 % lubricant; wherein the lubricant is magnesium stearate;
(d) from 0.5 % to 1 % glidant; wherein the glidant is colloidal silicon dioxide;
(e) 0 % to 10 % binder; where in the binder is selected from group consisting of povidone or hydroxypropyl methylcellulose;
(f) 0 % to 5 % disintegrant; wherein the disintegrant is selected from crospovidone, sodium starch glycolate and croscarmellose sodium; and
(g) 0 % to 2 % acidifying agent; wherein the acidifying agent is citric acid.

11. The immediate release pharmaceutical composition as claimed in claim 10, wherein said composition on a total of 100% by weight_comprises:
(a) from 2 % to 60 % 1-[(2-bromophenyl)sulfonyl]-5-methoxy-3-[(4-methyl-1-piperazinyl)methyl]-1H-indole dimesylate monohydrate;
(b) from 36 % to 97 % of microcrystalline cellulose;
(c) from 0.5 % to 2 % magnesium stearate;
(d) from 0.5 % to 1 % colloidal silicon dioxide;
(e) 0 % to 5 % povidone;
(f) 0 % to 4 % crospovidone; and
(g) 0 % to 2 % citric acid.

12. The immediate release pharmaceutical composition as claimed in claim 10 or claim 11, wherein the total weight of immediate release composition is from 100 mg to 600 mg.

## Patentansprüche

1. Pharmazeutische Zusammensetzung mit sofortiger Freisetzung, bei insgesamt 100 Gew.-% umfassend:
a) 2% bis 60% 1-[(2-Bromphenyl)sulfonyl]-5-methoxy-3-[(4-methyl-1-piperazinyl)methyl]-1H-indol oder ein pharmazeutisch annehmbares Salz davon;
b) 36 % bis 97 % Verdünnungsmittel oder insgesamt zwei Verdünnungsmittel; wobei das Verdünnungsmittel ausgewählt ist aus der Gruppe bestehend aus mikrokristalliner Cellulose, Lactosemonohydrat, zweibasischem Calciumphosphat, Lactose, Lactosehydrat, Lactoseanhydrat, Mannit, Stärke und Isomalt;
c) 0,5 % bis 2 % Schmiermittel; wobei das Schmiermittel Magnesiumstearat ist;
d) 0,5 % bis 1 % Gleitmittel; wobei das Gleitmittel kolloidales Siliciumdioxid ist; e) 0 % bis 10 % Bindemittel; wobei das Bindemittel ausgewählt ist aus der Gruppe bestehend aus Povidon oder Hydroxypropylmethylcellulose;
f) 0 % bis 5 % Sprengmittel; wobei das Sprengmittel ausgewählt ist aus Crospovidon, Natriumstärkeglykolat und Croscarmellose-Natrium; und
g) 0 % bis 2 % Säuerungsmittel; wobei das Säuerungsmittel Zitronensäure ist.

2. Pharmazeutische Zusammensetzung mit sofortiger Freisetzung nach Anspruch 1, wobei die Zusammensetzung bei insgesamt 100 Gew.-% Folgendes umfasst:
(a) 2% bis 60% 1-[(2-Bromphenyl)sulfonyl]-5-methoxy-3-[(4-methyl-1-piperazinyl)methyl]-1H-indoldimesylatmonohydrat;
(b) 36 % bis 97 % eines Verdünnungsmittels oder insgesamt zwei Verdünnungsmittel; wobei das Verdünnungsmittel ausgewählt ist aus der Gruppe bestehend aus mikrokristalliner Cellulose, Lactosemonohydrat, zweibasischem Calciumphosphat, Lactose, Lactosehydrat, Lactoseanhydrat, Mannit, Stärke und Isomalt;
(c) 0,5 % bis 2 % Schmiermittel; wobei das Schmiermittel Magnesiumstearat ist;
(d) 0,5 % bis 1 % Gleitmittel; wobei das Gleitmittel kolloidales Siliciumdioxid ist;
(e) 0 % bis 10 % Bindemittel; wobei das Bindemittel ausgewählt ist aus Gruppe bestehend aus Povidon oder Hydroxypropylmethylcellulose;
(f) 0 % bis 5 % Sprengmittel; wobei das Sprengmittel ausgewählt ist aus Crospovidon, Natriumstärkeglykolat und Croscarmellose-Natrium, und
(g) 0 % bis 2 % Säuerungsmittel; wobei das Säuerungsmittel Zitronensäure ist.

3. Pharmazeutische Zusammensetzungen mit sofortiger Freisetzung nach Anspruch 1 oder Anspruch 2, wobei die Zusammensetzung bei insgesamt 100 Gew.-% ausgewählt ist aus der Gruppe bestehend aus:
1) (a) 2 % bis 3 % 1-[(2-Bromphenyl)sulfonyl]-5-methoxy-3-[(4-methyl-1-piperazinyl)methyl]-1H-indoldimesylatmonohydrat, (b) 95 % bis 97 % Verdünnungsmittel, (c) 1 % Schmiermittel, und (d) 0,5 % Gleitmittel;
2) (a) 11 % bis 38 % 1-[(2-Bromphenyl)sulfonyl]-5-methoxy-3-[(4-methyl-1-piperazinyl)methyl]-1H-indoldimesylatmonohydrat, (b) 61 % bis 87 % eines Verdünnungsmittels oder insgesamt zwei Verdünnungsmittel, (c) 1 % Schmiermittel, (d) 0,5 % Gleitmittel, (e) 2 % Sprengmittel, und (f) 1 % Säuerungsmittel;
3) (a) 24 % bis 38 % 1-[(2-Bromphenyl)sulfonyl]-5-methoxy-3-[(4-methyl-1-piperazinyl)methyl]-1H-indoldimesylatmonohydrat (b) 61 % bis 72 % eines Verdünnungsmittels oder insgesamt zwei Verdünnungsmittel, (c) 1 % bis 1,25 % Schmiermittel, (d) 0,5 % Gleitmittel, und (e) 2 % Sprengmittel;
4) (a) 37 % bis 51 % 1-[(2-Bromphenyl)sulfonyl]-5-methoxy-3-[(4-methyl-1-piperazinyl)methyl]-1H-indoldimesylatmonohydrat, (b) 45 % bis 60 % Verdünnungsmittel, (c) 1 % Schmiermittel, (d) 0,5 % Gleitmittel, (e) 2 % Sprengmittel und (f) 1 % Säuerungsmittel;
5) (a) 36 % bis 60 % 1-[(2-Bromphenyl)sulfonyl]-5-methoxy-3-[(4-methyl-1-piperazinyl)methyl]-1H-indoldimesylatmonohydrat, (b) 36 % bis 62 % Verdünnungsmittel, (c) 0,5 % bis 1 % Schmiermittel, (d) 0,5 % Gleitmittel und (e) 2 % Sprengmittel; und
6) (a) 11 % bis 38 % 1-[(2-Bromphenyl)sulfonyl]-5-methoxy-3-[(4-methyl-1-piperazinyl)methyl]-1H-indoldimesylatmonohydrat; (b) 61 % bis 72 % Verdünnungsmittel; (c) 2 % bis 5 % Bindemittel; (d) 1 % Schmiermittel; (e) 0,5 % Gleitmittel und (f) 2 % bis 4 % Sprengmittel.

4. Pharmazeutische Zusammensetzung mit sofortiger Freisetzung nach einem der Ansprüche 1 bis 3, wobei die Dosierung von 1-[(2-Bromphenyl)sulfonyl]-5-methoxy-3-[(4-methyl-1-piperazinyl)methyl]-1H-indol oder einem pharmazeutisch annehmbaren Salz davon zwischen 5 mg und 200 mg beträgt.

5. Pharmazeutische Zusammensetzung mit sofortiger Freisetzung nach einem der Ansprüche 1 bis 4, wobei die Zusammensetzung in der Form einer Tablette oder Kapsel ist.

6. Pharmazeutische Zusammensetzung mit sofortiger Freisetzung nach einem der Ansprüche 1 bis 3, wobei die Zusammensetzung Folgendes aufweist:
i) weniger als 0,5 % Chlorverunreinigung;
ii) weniger als 0,5 % unbekannte Verunreinigungen;
iii) weniger als 1 % Gesamtverunreinigung.

7. Pharmazeutische Zusammensetzung mit sofortiger Freisetzung nach einem der Ansprüche 1 bis 3, wobei die Reinheit von 1-[(2-Bromphenyl)sulfonyl]-5-methoxy-3-[(4-methyl-1-piperazinyl)methyl]-1H-indoldimesylatmonohydrat 99,3 % beträgt.

8. Pharmazeutische Zusammensetzung mit sofortiger Freisetzung nach einem der Ansprüche 1 bis 3, wobei die Zusammensetzung Folgendes aufweist:
i) 99,3 % Reinheit von 1-[(2-Bromphenyl)sulfonyl]-5-methoxy-3-[(4-methyl-1-piperazinyl)methyl]-1H-indoldimesylatmonohydrat;
ii) weniger als 0,5 % Chlorverunreinigung;
iii) weniger als 0,5 % unbekannte Verunreinigungen;
iv) weniger als 1 % Gesamtverunreinigung.

9. Pharmazeutische Zusammensetzung mit sofortiger Freisetzung nach einem der Ansprüche 1 bis 3, wobei das 1-[(2-Bromphenyl)sulfonyl]-5-methoxy-3-[(4-methyl-1-piperazinyl)methyl]-1H-indoldimesylatmonohydrat innerhalb von 30 Minuten von 85 % bis 100 % freigesetzt wird, wenn es mit dem rotierenden Paddel bei 100 U/min mit 900 ml Auflösungsmedium, 01N Salzsäure oder Wasser bei 37 °C getestet wird.

10. Pharmazeutische Zusammensetzung mit sofortiger Freisetzung nach Anspruch 5, wobei die Zusammensetzung mit insgesamt 100 Gew.-% Folgendes umfasst:
(a) 2 % bis 60 % 1-[(2-Bromphenyl)sulfonyl]-5-methoxy-3-[(4-methyl-1-piperazinyl)methyl]-1H-indoldimesylatmonohydrat;
(b) 36 % bis 97 % eines Verdünnungsmittels oder insgesamt zwei Verdünnungsmittel; wobei das Verdünnungsmittel ausgewählt ist aus der Gruppe bestehend aus mikrokristalliner Cellulose, Lactosemonohydrat, zweibasischem Calciumphosphat, Lactose, Lactosehydrat, Lactoseanhydrat, Mannit, Stärke und Isomalt;
(c) 0,5 % bis 2 % Schmiermittel; wobei das Schmiermittel Magnesiumstearat ist;
(d) 0,5 % bis 1 % Gleitmittel; wobei das Gleitmittel kolloidales Siliciumdioxid ist;
(e) 0 % bis 10 % Bindemittel; wobei das Bindemittel ausgewählt ist aus Gruppe bestehend aus Povidon oder Hydroxypropylmethylcellulose;
(f) 0 % bis 5 % Sprengmittel; wobei das Sprengmittel ausgewählt ist aus Crospovidon, Natriumstärkeglykolat und Croscarmellose-Natrium; und
(g) 0 % bis 2 % Säuerungsmittel; wobei das Säuerungsmittel Zitronensäure ist.

11. Pharmazeutische Zusammensetzung mit sofortiger Freisetzung nach Anspruch 10, wobei die Zusammensetzung bei insgesamt 100 Gew.-% Folgendes umfasst:
(a) 2 % bis 60 % 1-[(2-Bromphenyl)sulfonyl]-5-methoxy-3-[(4-methyl-1-piperazinyl)methyl]-1H-indoldimesylatmonohydrat;
(b) 36 % bis 97 % mikrokristalline Cellulose;
(c) 0,5 % bis 2 % Magnesiumstearat;
(d) 0,5 % bis 1 % kolloidales Siliciumdioxid;
(e) 0 % bis 5 % Povidon;
(f) 0 % bis 4 % Crospovidon; und
(g) 0 % bis 2 % Zitronensäure.

12. Pharmazeutische Zusammensetzung mit sofortiger Freisetzung nach Anspruch 10 oder Anspruch 11, wobei das Gesamtgewicht der Zusammensetzung mit sofortiger Freisetzung 100 mg bis 600 mg beträgt.

## Revendications

1. Composition pharmaceutique à libération immédiate sur un total de 100 % en poids comprenant :
a) de 2 % à 60 % de 1-[(2-bromophényl)sulfonyl]-5-méthoxy-3-[(4-méthyl-1-pipérazinyl)méthyl]-1H-indole ou d'un sel pharmaceutiquement acceptable de celui-ci ;
b) de 36 % à 97 % d'un diluant ou d'un total de deux diluants ; ledit diluant étant choisi dans le groupe constitué par la cellulose microcristalline, le monohydrate de lactose, le phosphate de calcium dibasique, le lactose, un hydrate de lactose, un anhydrate de lactose, le mannitol, l'amidon et l'isomalt ;
c) de 0,5 % à 2 % d'un lubrifiant ; ledit lubrifiant étant le stéarate de magnésium ;
d) de 0,5 % à 1 % d'un agent de glissement ; ledit agent de glissement étant le dioxyde de silicium colloïdal ;
e) 0 % à 10 % d'un liant; ledit liant étant choisi dans le groupe constitué par la povidone ou l'hydroxypropylméthylcellulose ;
f) 0 % à 5 % d'un délitant ; ledit délitant étant choisi parmi la crospovidone, le glycolate d'amidon sodique et le sodium de croscarmellose ; et
g) 0 % à 2 % d'un agent acidifiant ; ledit agent acidifiant étant l'acide citrique.

2. Composition pharmaceutique à libération immédiate selon la revendication 1, ladite composition comprenant sur un total de 100 % en poids :
(a) de 2 % à 60 % de monohydrate de dimésylate de 1-[(2-bromophényl)sulfonyl]-5-méthoxy-3-[(4-méthyl-1-pipérazinyl)méthyl]-1H-indole ;
(b) de 36 % à 97 % d'un diluant ou d'un total de deux diluants ; ledit diluant étant choisi dans le groupe constitué par la cellulose microcristalline, le monohydrate de lactose, le phosphate de calcium dibasique, le lactose, un hydrate de lactose, un anhydrate de lactose, le mannitol, l'amidon et l'isomalt ;
(c) de 0,5 % à 2 % d'un lubrifiant ; ledit lubrifiant étant le stéarate de magnésium ;
(d) de 0,5 % à 1 % d'un agent de glissement ; ledit agent de glissement étant le dioxyde de silicium colloïdal ;
(e) 0 % à 10 % d'un liant; ledit liant étant choisi dans le groupe constitué par la povidone ou l'hydroxypropylméthylcellulose ;
(f) 0 % à 5 % d'un délitant ; ledit délitant étant choisi parmi la crospovidone, le glycolate d'amidon sodique et le sodium de croscarmellose et
(g) 0 % à 2 % d'un agent acidifiant ; ledit agent acidifiant étant l'acide citrique.

3. Compositions pharmaceutiques à libération immédiate selon la revendication 1 ou la revendication 2, ladite composition sur un total de 100 % en poids étant choisie dans le groupe constitué par :
1) (a) de 2 % à 3 % de monohydrate de dimésylate de 1-[(2-bromophényl)sulfonyl]-5-méthoxy-3-[(4-méthyl-1-pipérazinyl)méthyl]-1H-indole, (b) de 95 % à 97 % d'un diluant, (c) 1 % d'un lubrifiant et (d) 0,5 % d'un agent de glissement ;
2) (a) de 11 % à 38 % de monohydrate de dimésylate de 1-[(2-bromophényl)sulfonyl]-5-méthoxy-3-[(4-méthyl-1-pipérazinyl)méthyl]-1H-indole, (b) de 61 % à 87 % d'un diluant ou d'un total de deux diluants, (c) 1 % d'un lubrifiant, (d) 0,5 % d'un agent de glissement, (e) 2 % d'un délitant et (f) 1 % d'un agent acidifiant ;
3) (a) de 24 % à 38 % de monohydrate de dimésylate de 1-[(2-bromophényl)sulfonyl]-5-méthoxy-3-[(4-méthyl-1-pipérazinyl)méthyl]-1H-indole, (b) de 61 % à 72 % d'un diluant ou d'un total de deux diluants, (c) de 1 % à 1,25 % d'un lubrifiant, (d) 0,5 % d'un agent de glissement et (e) 2 % d'un délitant ;
4) (a) de 37 % à 51 % de monohydrate de dimésylate de 1-[(2-bromophényl)sulfonyl]-5-méthoxy-3-[(4-méthyl-1-pipérazinyl)méthyl]-1H-indole, (b) de 45 % à 60 % d'un diluant, (c) 1 % d'un lubrifiant, (d) 0,5 % d'un agent de glissement, (e) 2 % d'un délitant et (f) 1 % d'un agent acidifiant ;
5) (a) de 36 % à 60 % de monohydrate de dimésylate de 1-[(2-bromophényl)sulfonyl]-5-méthoxy-3-[(4-méthyl-1-pipérazinyl)méthyl]-1H-indole, (b) de 36 % à 62 % d'un diluant, (c) de 0,5 % à 1 % d'un lubrifiant, (d) 0,5 % d'un agent de glissement et (e) 2 % d'un délitant ; et
6) (a) de 11 % à 38 % de monohydrate de dimésylate de 1-[(2-bromophényl)sulfonyl]-5-méthoxy-3-[(4-méthyl-1-pipérazinyl)méthyl]-1H-indole ; (b) de 61 % à 72 % d'un diluant ; (c) de 2 % à 5 % d'un liant ; (d) 1 % d'un lubrifiant ; (e) 0,5 % d'un agent de glissement et (f) de 2 % à 4 % d'un délitant.

4. Composition pharmaceutique à libération immédiate selon l'une quelconque des revendications 1 à 3, le dosage du 1-[(2-bromophényl)sulfonyl]-5-méthoxy-3-[(4-méthyl-1-pipérazinyl)méthyl]-1H-indole ou d'un sel pharmaceutiquement acceptable de celui-ci étant de 5 mg à 200 mg.

5. Composition pharmaceutique à libération immédiate selon l'une quelconque des revendications 1 à 4, ladite composition étant sous la forme d'un comprimé ou d'une capsule.

6. Composition pharmaceutique à libération immédiate selon l'une quelconque des revendications 1 à 3, ladite composition comportant :
i) moins de 0,5 % d'impuretés chlorées ;
ii) moins de 0,5 % d'impuretés inconnues ;
iii) moins de 1 % d'impuretés totales.

7. Composition pharmaceutique à libération immédiate selon l'une quelconque des revendications 1 à 3, la pureté du monohydrate de dimésylate de 1-[(2-bromophényl)sulfonyl]-5-méthoxy-3-[(4-méthyl-1-pipérazinyl)méthyl]-1H-indole étant de 99,3 %.

8. Composition pharmaceutique à libération immédiate selon l'une quelconque des revendications 1 à 3, ladite composition comportant :
i) une pureté de 99,3 % du monohydrate de dimésylate de 1-[(2-bromophényl)sulfonyl]-5-méthoxy-3-[(4-méthyl-1-pipérazinyl)methyl]-1H-indole ;
ii) moins de 0,5 % d'impuretés chlorées ;
iii) moins de 0,5 % d'impuretés inconnues ;
iv) moins de 1 % d'impuretés totales.

9. Composition pharmaceutique à libération immédiate selon l'une quelconque des revendications 1 à 3, de 85 % à 100 % dudit monohydrate de dimésylate de 1-[(2-bromophényl)sulfonyl]-5-méthoxy-3-[(4-méthyl-1-pipérazinyl)méthyl]-1H-indole étant libéré dans un délai de 30 minutes lorsqu'il est testé avec une pale rotative à 100 tours par minute avec 900 ml d'un milieu de dissolution, de l'acide chlorhydrique 01 N ou de l'eau à 37°C.

10. Composition pharmaceutique à libération immédiate selon la revendication 5, ladite composition sur un total de 100 % en poids comprenant :
(a) de 2 % à 60 % de monohydrate de dimésylate de 1-[(2-bromophényl)sulfonyl]-5-méthoxy-3-[(4-méthyl-1-pipérazinyl)méthyl]-1H-indole ;
(b) de 36 % à 97 % d'un diluant ou d'un total de deux diluants ; ledit diluant étant choisi dans le groupe constitué par la cellulose microcristalline, le monohydrate de lactose, le phosphate de calcium dibasique, le lactose, un hydrate de lactose, un anhydrate de lactose, le mannitol, l'amidon et l'isomalt ;
(c) de 0,5 % à 2 % d'un lubrifiant ; ledit lubrifiant étant le stéarate de magnésium ;
(d) de 0,5 % à 1 % d'un agent de glissement ; ledit agent de glissement étant le dioxyde de silicium colloïdal ;
(e) 0 % à 10 % d'un liant; ledit liant étant choisi dans le groupe constitué par la povidone ou l'hydroxypropylméthylcellulose ;
(f) 0 % à 5 % d'un délitant ; ledit délitant étant choisi parmi la crospovidone, le glycolate d'amidon sodique et le sodium de croscarmellose ; et
(g) 0 % à 2 % d'un agent acidifiant ; ledit agent acidifiant étant l'acide citrique.

11. Composition pharmaceutique à libération immédiate selon la revendication 10, ladite composition sur un total de 100 % en poids comprenant :
(a) de 2 % à 60 % de monohydrate de dimésylate de 1-[(2-bromophényl)sulfonyl]-5-méthoxy-3-[(4-méthyl-1-pipérazinyl)méthyl]-1H-indole ;
(b) de 36 % à 97 % de cellulose microcristalline ;
(c) de 0,5 % à 2 % de stéarate de magnésium ;
(d) de 0,5 % à 1 % de dioxyde de silicium colloïdal ;
(e) 0 % à 5 % de povidone ;
(f) 0 % à 4 % de crospovidone ; et
(g) 0 % à 2 % d'acide citrique.

12. Composition pharmaceutique à libération immédiate selon la revendication 10 ou la revendication 11, le poids total de la composition à libération immédiate étant de 100 mg à 600 mg.
